# EUROPEAN PATENT APPLICATION

(11) **EP 4 494 603 A1**
(43) Date of publication of application: **22.01.2025**
(21) Application number: 23825779.4
(22) Date of filing: 10.02.2023
(51) Int. Cl.: A61F 2/24

(54) **ARTIFICIAL HEART VALVE**

(30) Priority: 24.06.2022 CN 202210738715
(71) Applicant: Jiangsu Trulive Medtech Co., Ltd., Nantong, Jiangsu 226400 (CN)
(72) Inventor: YANG, Wei, Shanghai 201206 (CN); WEI, Yongqiang, Shanghai 201206 (CN)
(74) Representative: Nordmeyer, Philipp Werner
(86) International application number: PCT/CN2023/075388
(87) International publication number: WO 2023/246120

(57) **Abstract**

The present invention provides an artificial heart valve including a stent and a prosthetic leaflet. The stent includes a mesh and a first channel wall joined to the stent. The first channel wall delimits a first channel for receiving a portion of the prosthetic leaflet therein. The prosthetic leaflet can be secured to the stent by being inserted through the first channel and attached to the first channel wall. In this artificial heart valve, the prosthetic leaflet can be uniformly secured to stent, with fewer wrinkles being formed therein. This allows uniform stressing of the leaflet and hence improved post-implantation durability and anti-calcification performance of the artificial heart valve.

## Description

### TECHNICAL FIELD

The present invention relates to the field of medical devices, and particularly to an artificial heart valve.

### BACKGROUND

With the development of social economy and the aging of population, senile valvular disease and valvular disease caused by coronary artery disease and myocardial infarction are becoming increasingly common. Studies have found over 13.3% with varying degrees of valvular heart disease among those 75 years or older. Therefore, heart valve disease has become one of the major threats to human health.

The human heart is divided into four chambers: the left atrium, the left ventricle, the right atrium and the right ventricle. The atria are separated from the ventricles by the atrioventricular valves, each consisting of leaflets, an annulus, chordae tendineae and papillary muscles. The leaflets can open and close to function as a one-way valve which allows blood flow only from the atrium to the respective ventricle. The atrioventricular valve that separates the left atrium from the left ventricle is called the mitral valve, and the atrioventricular valve that separates the right atrium from the right ventricle is called the tricuspid valve.

Compared with traditional surgery, transcatheter valve replacement is advantageous in no need for thoracotomy, less trauma and faster patient recovery, among others, and therefore has received widespread attention from experts and scholars. However, following the deployment of a replacement artificial heart valve, stress tends to concentrate at wrinkles in its prosthetic leaflets from blood flow over time, and the prosthetic leaflets may be therefore torn or otherwise damaged over time. Therefore, such wrinkles are detrimental to the durability of the artificial heart valve. Moreover, wrinkled prosthetic leaflets are associated with an increased risk of calcification.

### SUMMARY

It is an objective of the present invention to provide an artificial heart valve, in which a prosthetic leaflet can be uniformly attached to a stent with fewer wrinkles being formed therein and can thereby be more uniformly stressed, imparting better post-implantation durability and anti-calcification performance to the artificial heart valve.

The above objective is attained by an artificial heart valve provided in the present invention, which includes a stent and a prosthetic leaflet. The stent includes a mesh and a first channel wall joined to the mesh. The first channel wall delimits a first channel for receiving a portion of the prosthetic leaflet therein. The prosthetic leaflet is able to be secured to the stent by being inserted through the first channel and attached to the first channel wall.

Optionally, the prosthetic leaflet may be able to be inserted through the first channel and folded and to be sutured to the first channel wall using a suture.

Optionally, the stent may define inflow and outflow ends opposing each other along an axis of the stent, wherein after being inserted through the first channel, the prosthetic leaflet is able to be folded toward the inflow end, or toward the outflow end.

Optionally, the first channel may be arranged circumferentially around the stent and match the prosthetic leaflet in shape.

Optionally, the first channel may be curved and symmetrical about an axis of the stent and may have a width of 0.1 mm to 1 mm measured perpendicularly to its own extension direction.

Optionally, the stent may further include second channel walls joined to the mesh, the second channel walls delimiting second channels for receiving portions of the prosthetic leaflet therein, which are able to be inserted through the second channels and sutured to the second channel walls.

Optionally, the prosthetic leaflet may include protruding portions, notches, a curved portion and a flat portion, the number of the protruding portions and the notches is both two, the two protruding portions contiguous with opposite ends of the flat portion, the two notches contiguous with opposite ends of the curved portion, each of the notches contiguous with a respective one of the protruding portions,
the curved portion configured to be inserted through the first channel and sutured to the first channel wall, the protruding portions configured to be inserted through the second channels and sutured to the second channel walls.

Optionally, the second channels may extend along an axis of the stent, the second channels have a length of 1 mm to 10 mm measured in an extension direction of the second channels, and the second channels have a width of 0.1 mm to 2 mm measured perpendicularly to the extension direction of the second channels.

Optionally, the prosthetic leaflet may be sutured to the stent on an outer circumference of the stent.

Optionally, the suture may include a suture body and two knot tails at respective opposite ends of the suture body, wherein when the prosthetic leaflet is sutured to the stent, the suture body and the knot tails are all located on the outer circumference of the stent.

Optionally, the prosthetic leaflet may be provided therein with a plurality of suture holes arranged in a circumferential direction of the prosthetic leaflet,
wherein when the prosthetic leaflet is sutured to the first channel wall, the suture is successively threaded through each of the suture holes and each time after the suture is threaded through one of the suture holes, the suture is connected to the first channel wall.

Optionally, at least two sutures may be used, each of which is threaded through a predetermined number of suture holes.

Optionally, all the suture holes may be distributed uniformly in the prosthetic leaflet, and all the suture holes are in symmetry in the prosthetic leaflet.

Optionally, the prosthetic leaflet may include a curved portion configured to be inserted through the first channel and sutured to the first channel wall, wherein adjacent suture holes are spaced at an interval of 0.1 mm to 3 mm and the suture holes are spaced at a distance of 0.1 mm to 5 mm from an edge of the curved portion and have a diameter of 0.1 mm to 2 mm.

The present invention provides an artificial heart valve including a stent and a prosthetic leaflet. The stent includes a mesh and a first channel wall joined to the mesh. The first channel wall delimits a first channel for receiving a portion of the prosthetic leaflet therein. The prosthetic leaflet is able to be secured to the stent by being inserted through the first channel and attached to the first channel wall. With this arrangement, the prosthetic leaflet can be uniformly attached to the stent by being secured to the first channel wall of the stent. In this way, fewer wrinkles will be formed in the secured prosthetic leaflet, making the prosthetic leaflet more uniformly stressed by blood flow and reducing the risk of stress concentration developing in the prosthetic leaflet. Therefore, post-implantation durability and anti-calcification performance of the artificial heart valve can be enhanced.

In the artificial heart valve of the present invention, part of the prosthetic leaflet can be inserted through the first channel and sutured to the first channel wall at the outer circumference of the stent. This can effectively prevent stress concentration from developing around stitch holes in the prosthetic leaflet, additionally enhancing uniformity of stressing of the prosthetic leaflet and post-implantation durability and anti-calcification performance of the artificial heart valve.

### BRIEF DESCRIPTION OF THE DRAWINGS

Fig. 1 shows a simplified schematic structural view of an artificial heart valve according to a preferred embodiment of the present invention, in which the dashed line represents an axis of symmetry of a prosthetic leaflet.
Fig. 2 shows a schematic partial structural view of a stent according to a preferred embodiment of the present invention, taken from a first perspective.
Fig. 3 shows a simplified schematic top view of an artificial heart valve according to a preferred embodiment of the present invention.
Fig. 4 shows a schematic partial axial cross-sectional view of an artificial heart valve according to a preferred embodiment of the present invention.
Fig. 5 shows a schematic partial structural view of a stent according to a preferred embodiment of the present invention, taken from a second perspective.
Fig. 6 shows a schematic structural view of a prosthetic leaflet according to a preferred embodiment of the present invention.
Fig. 7 shows a schematic partial structural view of an artificial heart valve according to a preferred embodiment of the present invention, taken from the first perspective.
Fig. 8 shows a schematic partial structural view of an artificial heart valve according to a preferred embodiment of the present invention, taken from the second perspective.
Fig. 9 shows a simplified schematic top view of a prosthetic leaflet according to a preferred embodiment of the present invention, which is sutured using a single suture.
Fig. 10 shows a schematic enlarged partial view of a prosthetic leaflet according to another preferred embodiment of the present invention, which is sutured using multiple sutures each with two knot tails being formed on opposite sides of a single middle suture hole.
Fig. 11 shows a schematic enlarged partial view of a prosthetic leaflet according to a further preferred embodiment of the present invention, which is sutured using multiple sutures each with two knot tails being formed on opposite sides of two middle suture holes.

### List of Reference Numerals

1 stent; 11 mesh; 12 first channel wall; 13 first channel; 14 inflow end; 15 outflow end; 16 second channel wall; 17 second channel; 2 prosthetic leaflet; 21 suture hole; 22 protruding portion; 23 notch; 24 curved portion; 25 flat portion; 3 suture; 31 suture body; 32 knot tail; 4 skirt.

### DETAILED DESCRIPTION

The present invention will be described in greater detail below with reference to the accompanying drawings, which illustrate specific embodiments thereof. From the following description, advantages and features of the invention will become apparent. Note that the figures are provided in a very simplified form not necessarily drawn to exact scale for the only purpose of helping to explain the disclosed embodiments in a more convenient and clearer way.

Directional and positional relationships described by the terms "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. are based on the orientations shown in the figures. They are intended merely to facilitate and simplify the explanation of the invention and do not indicate or imply that the stated components or elements have to assume, or be constructed or operated in, particular orientations. Therefore, they are not to be construed as limiting the invention.

As used herein, unless otherwise expressly specified or defined, the terms "mounting", "connecting", "securing" and grammatical variants thereof should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. As used herein, the term "plurality" means at least two, for example, two, three or more.

The present invention will be described in detail below with respect to the accompanying drawings, which illustrate preferred embodiments thereof. If there is no conflict, the embodiments described below and features thereof can complement or be combined with each other.

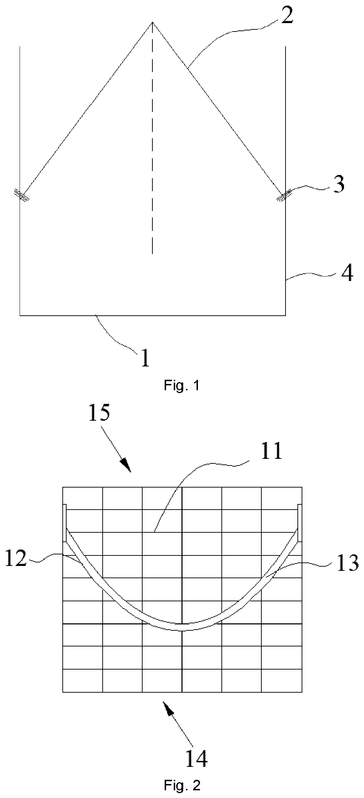

Referring to Figs. 1-2, a preferred embodiment of the present invention provides an artificial heart valve, the artificial heart valve is used to connect with the native annulus and replace the natural mitral or tricuspid valve. The artificial heart valve includes a stent 1 and a prosthetic leaflet 2. The stent 1 includes a mesh 11 and a first channel wall 12 joined to the mesh. The first channel wall 12 delimits a first channel 13 for receiving a portion of the prosthetic leaflet 2 therein. The prosthetic leaflet 2 can be inserted through the first channel 13 and attached to the first channel wall 12. In this way, the prosthetic leaflet 2 can be secured to the stent 1.

Conventionally, the prosthetic leaflet 2 of the artificial heart valve would have been directly sutured to the mesh 11 of the stent 1 as a common practice. However, in this approach, the structure of the mesh 11 may not allow the suturing of the prosthetic leaflet 2 to occur at desired locations and some wrinkles may be formed in the prosthetic leaflet 2 after the prosthetic leaflet 2 is secured to the stent 1. When the artificial heart valve is implanted into a human body with such wrinkles, the prosthetic leaflet 2 may be stressed non-uniformly by blood flow and local stress concentration may occur. Consequently, the prosthetic leaflet 2 may be damaged or develop calcification over time.

The prosthetic leaflet 2 provided by the present invention can be fixedly connected to the first channel wall 12 of the stent 1, and can achieve uniform fixation of the prosthetic leaflet 2 on the stent 1 through the connection between the prosthetic leaflet 2 and the adjacent first channel wall 12, thereby fewer wrinkles of the prosthetic leaflet 2 are formed after fixation. As a result, the prosthetic leaflet 2 can be more uniformly stressed and associated with a reduced risk of stress concentration. Therefore, post-implantation durability and anti-calcification performance of the artificial heart valve can be improved.

According to embodiments of the present application, the stent 1 can serve a number of functions for the artificial heart valve, including acting as a main structural element of the artificial heart valve, carrying the prosthetic leaflet 2, connecting with a delivery system, and so forth. The present application is not limited to any particular structure of the stent 1. For example, the stent 1 may be fabricated by braiding or laser cutting. The present application is also not limited to any particular material, from which the stent 1 is fabricated. For example, the stent 1 may be made of an elastic material such as a nickel-titanium alloy or other material with shape memory properties. The stent 1 may also be made of a titanium alloy, cobalt-chromium alloy, MP35N, 316 stainless steel or other material. Of course, the present application is not so limited, because the stent 1 may also be chosen as a biocompatible metal frame or laser-cut metal tube, or made of an elastically or plastically deformable material, such as a material which can be expanded using a balloon.

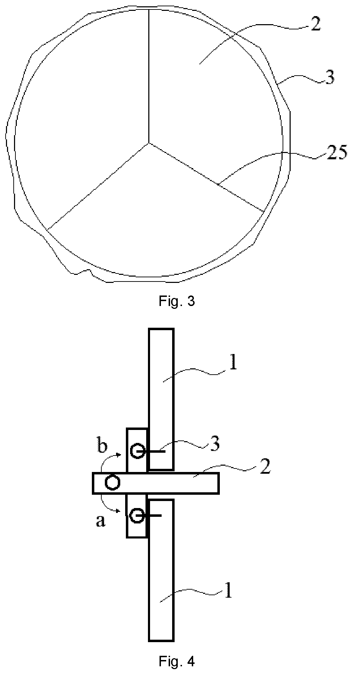

In addition, the prosthetic leaflet 2 may dynamically switch between open and closed configurations. In the closed configuration, the prosthetic leaflet 2 may tightly gather or close by sealing abutment. Optionally, two or three (three in the example of Fig. 3) prosthetic leaflets 2 may be provided.

The present invention is not limited to any particular material of the prosthetic leaflet 2. The prosthetic leaflet 2 may be formed of any suitable material or combination of materials. In some embodiments, the prosthetic leaflet 2 may be chosen as a biological tissue material, which is derived, for example, from animal heart valve tissue that has been chemically stabilized, or from animal pericardial tissue, such as bovine, ovine, porcine or equine pericardial tissue, with bovine pericardial tissue being preferred. In some alternative embodiments, the prosthetic leaflet 2 may also be made of small intestinal submucosal tissue. In further alternative embodiments, the prosthetic leaflet 2 may also be made of a synthetic material. Examples of the synthetic material may include expanded polytetrafluoroethylenes or polyesters. Optionally, examples of the synthetic material may also include thermoplastic polycarbonates polyurethanes, polyether-urethanes, segmented polyether-urethanes, silicone-polyether-urethanes, silicone polycarbonates polyurethanes, ultra-high molecular weight polyethylenes and combinations thereof. Preferably, examples of the synthetic material may also include biocompatible polymers such as polyolefins, elastomers, polyethylene glycols, polyethersulfones, polysulfones, polyvinylpyrrolidones, polyvinyl chlorides, fluoropolymers, silicone polyesters, siloxanes oligomers and siloxanes lactones, and combinations and block copolymers thereof.

The present application is also not limited to any particular shape of openings in the mesh 11 of the stent 1. For example, the openings in the mesh 11 may be square, rectangular, diamond-shaped or otherwise shaped.

Further, after being inserted through the first channel 13, the prosthetic leaflet 2 may be folded and sutured to the first channel wall 12 using a suture 3. This allows the prosthetic leaflet 2 to be sutured to the first channel wall 12 of the stent 1 over a larger area, ensuring secure attachment of the prosthetic leaflet 2 to the stent 1. In one embodiment, the folded portion of the prosthetic leaflet 2 that have been inserted through the first channel 13 may be sutured to the first channel wall 12. The present application is not limited to any particular material of the suture 3. For example, the suture 3 may be formed of polytetrafluoroethylene (PTFE), expanded PTFE (ePTFE), polyethylene (PE) or the like.

With continued reference to Fig. 1, the artificial heart valve further includes a skirt 4 disposed around the outer circumference of the stent 1. The skirt 4 may be sutured to the stent 1 using a suture 3. In one embodiment, the skirt 4 may be sutured to the outer circumference of the stent 1 before the prosthetic leaflet 2 is sutured to the stent 1. In an alternative embodiment, the skirt 4 may be sutured to the outer circumference of the stent 1 after the prosthetic leaflet 2 is sutured to the stent 1. The present application is not limited to any particular material of the skirt 4. The skirt 4 may be chosen, as required, as a piece of knitted, woven or braided fabric made of polyester, PTFE, ePTFE or another material.

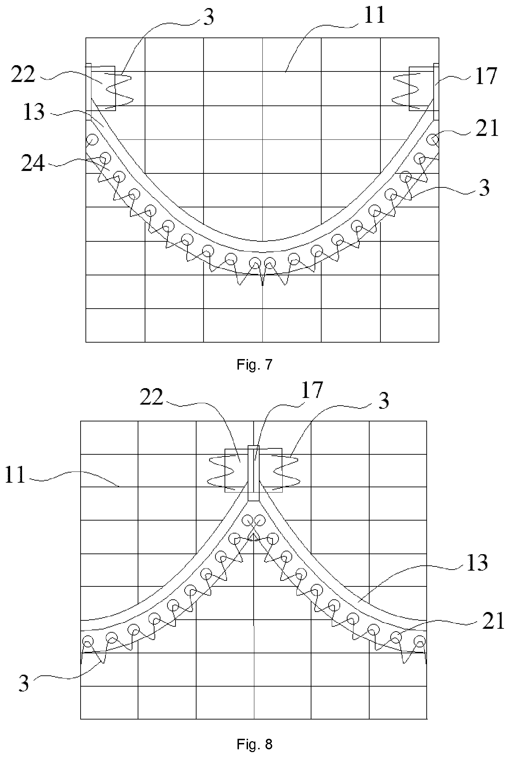

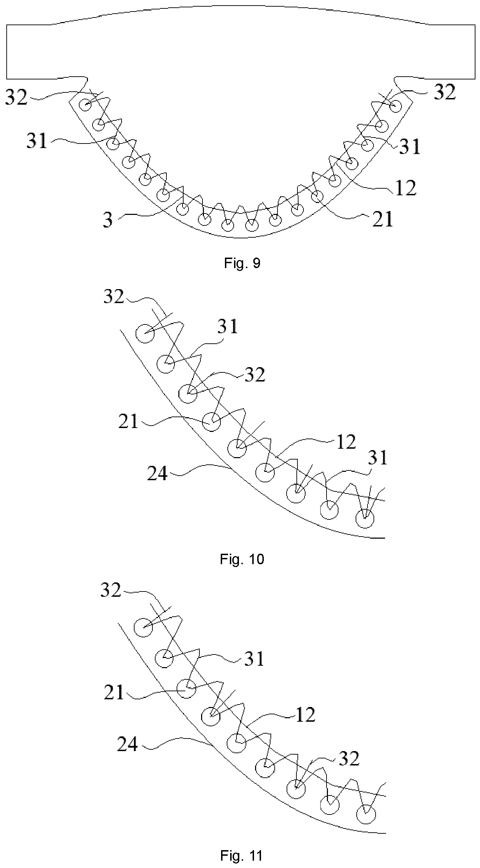

Referring to Fig. 2, the stent 1 defines an inflow end 14 and an outflow end 15, which axially oppose each other. In one embodiment, the prosthetic leaflet 2 may be inserted through the first channel 13 and then folded toward the inflow end 14 (see Fig. 7). In an alternative embodiment, the prosthetic leaflet 2 may be inserted through the first channel 13 and then folded toward the outflow end 15 (see Fig. 9). In greater detail, as shown in Fig. 4, in practical applications, after the prosthetic leaflet 2 is inserted through the first channel 13 of the stent 1, the prosthetic leaflet 2 may be folded toward the inflow end 14, i.e., in the direction labeled as a in the figure, or toward the outflow end 15, i.e., in the direction labeled as b in the figure. The specific folding direction of the prosthetic leaflet 2 can be set as needed.

Preferably, the first channel 13 is arranged circumferentially around the stent 1. In a preferred embodiment, the first channel 13 is arranged into a ring. In this case, the prosthetic leaflet 2 may be inserted through the first channel 13 and secured to the first channel wall 12 across the entire outer circumference of the prosthetic leaflet 2. In an alternative embodiment, the first channel 13 may form part of a ring. In this case, the prosthetic leaflet 2 may be inserted through the first channel 13 and secured to the first channel wall 12 across part of the outer circumference of the prosthetic leaflet 2.

Further, the first channel 13 matches the prosthetic leaflet 2 in shape so that, after being inserted through the first channel 13, the prosthetic leaflet 2 is located from the first channel wall 12 substantially at the same distance. This enables uniform attachment of the prosthetic leaflet 2 to the stent 1.

In one embodiment, the first channel 13 is curved, the first channel 13 is curved to the same curvature as the prosthetic leaflet 2 where it is attached to the stent 1. In this way, the prosthetic leaflet 2 may be so sutured that the suture 3 maintains a constant pattern across its total length. This can reduce wrinkles formed in the prosthetic leaflet 2 after it is sutured, allowing for more uniform post-implantation stressing of the prosthetic leaflet 2.

Referring to Fig. 2, in one embodiment, the first channel 13 is arranged in symmetry about an axis of the stent 1. This allows the first channel 13 to match the prosthetic leaflet 2 in shape, reducing wrinkles formed in the prosthetic leaflet 2. It will be appreciated that the "axis" of the stent 1 refers to a straight line running in an axial direction of the stent 1.

In addition, the first channel 13 may have a width of 0.1 mm to 1 mm measured perpendicularly to its own extension direction, which is line with a thickness of the prosthetic leaflet 2. Preferably, the width of the first channel 13 measured perpendicularly to the extension direction is 0.1 mm to 0.5 mm. This allows it to be suitably used with prosthetic leaflets 2 of various common sizes.

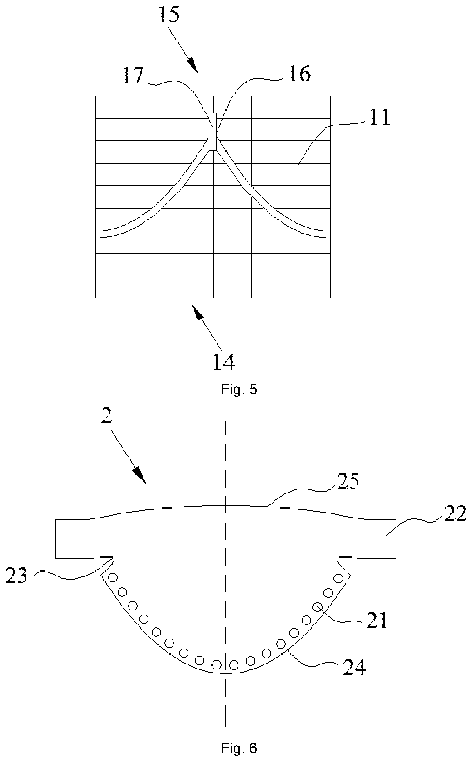

Referring to Fig. 5, the stent 1 further includes second channel walls 16 also joined to the mesh 11. The second channel walls 16 delimit second channels 17 for receiving portions of the prosthetic leaflet 2 therein. These portions of the prosthetic leaflet 2 can be inserted through the second channels 17 and sutured to the second channel walls 16. Suring the prosthetic leaflet 2 also to the second channel walls 16 allows it to be even more securely attached to the stent 1.

Referring to Fig. 6, in one embodiment, the prosthetic leaflet 2 includes protruding portions 22, notches 23, a curved portion 24 and a flat portion 25. The number of the protruding portions 22 and notches 23 is both two. The two protruding portions 22 are contiguous with opposite ends of the flat portion 25, and the two notches 23 are contiguous with opposite ends of the curved portion 24. Each of the notches 23 is contiguous with a respective one of the protruding portions 22. In other words, in the prosthetic leaflet 2, one of the protruding portions 22, the flat portion 25, the other protruding portion 22, one of the notches 23, the curved portion 24 and the other notch 23 are contiguous one with another sequentially in this order. The protruding portions 22 and the curved portion 24 can be considered as all extending from the notches 23 toward the outside of the prosthetic leaflet 2. That is, the prosthetic leaflet 2 can be considered as being recessed inwardly at the notches 23. Referring to Figs. 7 and 8, the curved portion 24 is configured to be inserted through the first channel 13 and secured to the first channel wall 12, and the protruding portions 22 are configured to be inserted through the second channels 17 and sutured to the second channel walls 16. With this arrangement, the second channels 17 enable the prosthetic leaflet 2 to be even more securely attached to the stent 1, preventing the prosthetic leaflet 2 from displacement under the stress from blood flow over time.

In one embodiment, the second channels 17 are aligned with the protruding portions 22 of the prosthetic leaflet 2, the second channels 17 match in shape with the protruding portions 22 of the prosthetic leaflet 2, thereby allowing the protruding portions 22 to be sutured to the second channel walls 16.

Additionally, the notches 23 are configured for abutment of junctions of the first channel wall 12 and the second channel walls 16 therein, which allows the prosthetic leaflet 2 to be secured to the stent 1 at defined locations. That is, the prosthetic leaflet 2 can be secured to the stent 1 so that the junctions of the first channel wall 12 and the second channel walls 16 abut them in their notches 23. This can prevent the prosthetic leaflet 2 from radially displacing outwards from the stent 1. It will be appreciated that, as used herein, the term "outwardly" refers to a direction radially away from the axis of the stent 1, while the term "inwardly" refers to a direction radially toward the axis of the stent 1.

In one embodiment, the protruding portions 22 are inserted through the second channels 17 and then sutured to the second channel walls 16 using sutures 3. Preferably, a length of extension of the protruding portions 22 beyond the prosthetic leaflet 2 (from the notches 23) is greater than a length of extension of the curved portion 24 beyond the prosthetic leaflet 2 (from the notches 23). This allows the protruding portions 22 to be sutured over a larger area, ensuring secure attachment of the prosthetic leaflet 2.

Referring to Fig. 3, the artificial heart valve may include multiple prosthetic leaflets 2, the prosthetic leaflet 2 may be closed when their flat portions 25 abut against one another. In case of the artificial heart valve being used for mitral valve replacement, it may have two prosthetic leaflets 2. When the artificial heart valve is used for tricuspid valve replacement, it may have three prosthetic leaflets 2 (see Fig. 3).

The present application is not limited to any particular shape of the flat portion 25 of the prosthetic leaflet 2, and possible shapes may include, but are not limited to, convex (i.e., curved away from the curved portion 24), concave (curved toward the curved portion 24) and straight shapes, with a convex shape being preferred because it allows the flat portions 25 of multiple prosthetic leaflets 2 to better sealingly abut against one another to achieve completely closure of the prosthetic leaflets 2.

In Fig. 6, the dashed line represents an axis of symmetry of the prosthetic leaflet 2, which is defined by a line connecting midpoints of the curved portion 24 and the flat portion 25. In one embodiment, the prosthetic leaflet 2 is symmetrical with respect to the connecting line, the two protruding portions 22 and the two notches 23 are both symmetrical with respect to the connecting line. This can additionally enhance the uniformity of post-implantation stressing of the prosthetic leaflet 2.

Referring to Figs. 5 and 8, the second channels 17 may extend axially with respect to the stent 1, and the protruding portions 22 may be vertically inserted into the second channels 17 and sutured to the second channel walls 16. In one embodiment, the second channels 17 are rectangular, the second channels 17 have a length of 1 mm to 10 mm measured in their extension direction, and the second channels 17 have a width of 0.1 mm to 2 mm measured perpendicularly to their extension direction. This allows them to accommodate prosthetic leaflets 2 of various shapes. Preferably, the length of the second channels 17 measured in their extension direction is 3 mm to 6 mm, and the width of the second channels 17 measured perpendicularly to their extension direction is 0.4 mm to 1 mm. This allows them to be suitably used with prosthetic leaflets 2 of various common sizes.

Referring to Fig. 3, in a preferred embodiment, the prosthetic leaflet 2 is sutured to the stent 1 on the outer circumference of the stent 1 using a suture 3, In this case, the suture 3 and stitch holes in the prosthetic leaflet 2 (i.e., openings left after the suture 3 is sewn through the prosthetic leaflet 2) are all located on an outer side of the stent 1. It will be appreciated that, here, the "outer side" of the stent 1 refers to its surface farther away from the axis of the stent 1, the "inner side" of the stent 1 refers to its surface closer to the axis of the stent 1.

After the artificial heart valve is implanted into a human body, the inner side of the stent 1 will be stressed by blood flow for a long time. Therefore, if the stitch holes in the prosthetic leaflet 2 are located on the inner side of the stent 1, the stitch holes will be exposed to such stress on the prosthetic leaflet 2, and stress concentration will readily tend to develop at the stitch holes over time. Consequently, the prosthetic leaflet 2 may be torn or calcified around the stitch holes.

According to the present invention, the prosthetic leaflet 2 is sutured to the stent 1 so that stitch holes formed in the prosthetic leaflet 2 are all located on the outer side of the stent 1 and the portion of the prosthetic leaflet 2 on the inner side of the stent 1 (which are to be stressed) is integral and continuous. In this way, stress concentration in the prosthetic leaflet 2 can be reduced or even avoided, allowing for more uniform stressing of the prosthetic leaflet 2 and reducing the risk of calcification of the prosthetic leaflet 2.

Of course, in other embodiments, the prosthetic leaflet 2 may also be sutured to the stent 1 on the inner circumference of the stent 1 using a suture 3. In such embodiments, the suture 3 and the stitch holes in the prosthetic leaflet 2 are all located on the inner side of the stent 1.

Referring to Figs. 6 to 8, in a preferred embodiment, the prosthetic leaflet 2 is provided with a plurality of suture holes 21 arranged in its circumferential direction. When the prosthetic leaflet 2 is sutured to the first channel wall 12, the suture 3 is threaded successively through each of the suture holes 21 and each time after the suture 3 is pulled out of a suture hole 21, it is looped around the first channel wall 12. In greater detail, the prosthetic leaflet 2 is sutured to the first channel wall 12 using a suture 3 in such a manner that each time after the suture 3 is threaded through a suture hole 21, it is looped around the first channel wall 12 and then threaded into the next adjacent suture hole 21. In this way, the prosthetic leaflet 2 can be sutured to the stent 1. This configuration ensures that when the prosthetic leaflet 2 is sutured on the stent I, the distance between the suture 3 passing through each position of the prosthetic leaflet 2 is equal. This allows the prosthetic leaflet 2 to be even more uniformly sutured to the stent 1, further reducing wrinkles formed in the prosthetic leaflet 2.

The present application is not limited to any particular method of forming the suture holes 21. The suture holes 21 may be formed in the prosthetic leaflet 2 using a laser cutting, stamping or other mechanical machining process. Preferably, the suture holes 21 are formed by laser cutting.

Referring to Figs. 9 to 11, the suture 3 may include a suture body 31 and two knot tails 32 at opposite ends of the suture body 31. The prosthetic leaflet 2 may be sutured to the stent 1 using such a suture 3 so that the suture body 31 and the knot tails 32 are all located on the outer circumference of the stent 1. Specifically, the suturing of the prosthetic leaflet 2 may involve: knotting a suture 3 to form a first knot tail 32 and threading the suture 3 into a suture hole 21 from the outside of the stent 1; threading the suture 3 through successively adjacent suture holes 21, wherein each time after the suture 3 is threaded through a suture hole 21, it is looped around the first channel wall 12, to which the prosthetic leaflet 2 is to be sutured; and finally, threading the suture 3 from the inner side to the outer side of the stent 1 and again knotting it to form another knot tail 32. In this way, the suture 3 (including the suture body 31 and the knot tails 32) and the stitch holes in the prosthetic leaflet 2 can be all kept on the outer side of the stent 1. This can reduce the risk of developing stress concentration in the prosthetic leaflet 2 under the stress of blood flow over time, which may cause damage to the prosthetic leaflet 2 or calcification thereof.

The prosthetic leaflet 2 may be sutured across its entire length, or across spaced portions thereof. In the former case, the prosthetic leaflet 2 may be sutured to the first channel wall 12 by threading a single suture 3 through the successively adjacent suture holes 21 in the prosthetic leaflet 2. In the latter case, the prosthetic leaflet 2 may be sutured to the first channel wall 12 using two or more sutures, each of the suture 3 sutures only a portion of the prosthetic leaflet 2 to the first channel wall 12.

When the prosthetic leaflet 2 is sutured to the stent 1 across its entire length using a single suture 3, once the suture 3 between the prosthetic leaflet 2 and the stent 1 breaks free, the prosthetic leaflet 2 will be completely separated from the stent 1, and the prosthetic leaflet 2 will fall off from the stent 1. In contrast, suturing portions of the prosthetic leaflet 2 can mitigate local buckling (i.e., wrinkling) that may occur during the suturing of the prosthetic leaflet 2, thus reducing or even eliminating non-uniform stressing of the prosthetic leaflet 2 that may be induced by such local buckling, extending the service life of the prosthetic leaflet 2 and lowering the risk of calcification of the prosthetic leaflet 2. Additionally, as the portions of the prosthetic leaflet 2 are sutured to the stent 1 using separate sutures 3, even if one of the sutures 3 breaks, only the corresponding portion of the prosthetic leaflet 2 will be separated from the stent 1, and the remaining portion(s) will not be affected at all and remain attached to the stent 1. Therefore, the prosthetic leaflet 2 can still serve its intended functions. In this way, the anti-calcification performance of the prosthetic leaflet 2 and overall durability of the prosthetic leaflet 2 can be greatly improved.

Preferably, the prosthetic leaflet 2 is sutured using at least two sutures 3, each of the sutures 3 is threaded through a predetermined number of suture holes 21. In other words, the suturing method for prosthetic leaflet 2 is spaced-portion suturing, where each suture 3 is only used to suture a predetermined number of suture holes 21 with the first channel wall 12. It will be appreciated that the predetermined number of suture holes 21, through which each suture 3 is threaded, is at least two. The actual number of suture holes 21 through which each suture 3 passes may be determined according to the requirements of practical applications.

In order to minimize the impact of breaking free of one or more sutures 3 on the attachment of the prosthetic leaflet 2 to the stent 1, the number of suture holes 21, through which each suture 3 is threaded, may be appropriately reduced to allow more smaller portions of the prosthetic leaflet 2 to be separately sutured.

Referring to Fig. 10, in a specific embodiment, each suture 3 may be threaded through three suture holes 21, wherein two knot tails 32 of the suture 3 are formed after it is pulled out of the respective outermost suture holes 21 on opposite sides of the remaining single middle suture hole 21. Referring to Fig. 11, in another specific embodiment, each suture 3 may be threaded through four suture holes 21, wherein two knot tails 32 of the suture 3 are formed after it is pulled out of the respective outermost suture holes 21 on opposite sides of the remaining two middle suture holes 21.

More preferably, the suture holes 21 in the prosthetic leaflet 2 are uniformly distributed, and the spacing between all suture holes 21 in the prosthetic leaflet 2 is equal. This allows the sutures 3 to be distributed on the prosthetic leaflet 2 with high uniformity, which provides for high positional accuracy of the prosthetic leaflet 2 sutured to the stent 1, fewer wrinkles formed in the sutured prosthetic leaflet 2, uniform post-implantation stressing of the prosthetic leaflet 2 and a reduced risk of the prosthetic leaflet 2 being torn.

Referring to Fig. 6, the suture holes 21 in the prosthetic leaflet 2 are distributed in symmetry. With this arrangement, the prosthetic leaflet 2 can be divided into two parts along an axis of symmetry, which are equally stressed, ensuring uniform stressing of the prosthetic leaflet 2.

Further, in order to ensure that the prosthetic leaflet 2 is able to withstand a certain level of stress from blood flow, adjacent suture holes 21 therein may be spaced at an interval of 0.1 mm to 3 mm. In addition, in order to facilitate suturing of the prosthetic leaflet 2 to the stent 1, the suture holes 21 therein may be spaced from an edge of the curved portion 24 at a distance of 0.1 mm to 5 mm, and the suture holes 21 may have a diameter of 0.1 mm to 2 mm. In a preferred embodiment, adjacent suture holes 21 are spaced at an interval of 0.5 mm to 1.5 mm, the suture holes 21 are spaced from the edge of the curved portion 24 at a distance of 1 mm to 3 mm, and the suture holes 21 have a diameter of 0.2 mm to 1 mm. These dimensions can accommodate prosthetic leaflets 2 of various common sizes.

In summary, in the artificial heart valve of the present invention, the prosthetic leaflet 2 can be uniformly attached to the stent 1 by being secured to the first channel wall 12 of the stent 1. This allows fewer wrinkles to be formed in the secured prosthetic leaflet 2, resulting in more uniform stressing of the prosthetic leaflet 2 by blood flow, a low risk of stress concentration in the prosthetic leaflet 2 and improved post-implantation durability and anti-calcification performance of the artificial heart valve.

In the artificial heart valve of the present invention, the prosthetic leaflet 2 can insert through the first channel 13, and the portion where the prosthetic leaflet 2 insert through can be sutured to the first channel wall 12 on the outer circumference of the stent 1. This can effectively prevent stress concentration from developing around stitch holes in the prosthetic leaflet 2, additionally enhancing uniformity of stressing of the prosthetic leaflet 2 and post-implantation durability of the artificial heart valve.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. An artificial heart valve comprising a stent and a prosthetic leaflet, wherein the stent comprises a mesh and a first channel wall joined to the mesh, the first channel wall delimiting a first channel for receiving a portion of the prosthetic leaflet therein, wherein the prosthetic leaflet is able to be inserted through the first channel and attached to the first channel wall, thereby securing the prosthetic leaflet to the stent.

2. The artificial heart valve of claim 1, wherein the prosthetic leaflet is able to be folded after being inserted through the first channel and is able to be sutured to the first channel wall using a suture.

3. The artificial heart valve of claim 2, wherein the stent defines an inflow end and an outflow end opposing each other along an axis of the stent, wherein after being inserted through the first channel, the prosthetic leaflet is able to be folded toward the inflow end, or toward the outflow end.

4. The artificial heart valve of claim 1, wherein the first channel is arranged circumferentially around the stent, and the first channel matches the prosthetic leaflet in shape.

5. The artificial heart valve of claim 1, wherein the first channel is curved, the first channel is symmetrical about an axis of the stent, and the first channel has a width of 0.1 mm to 1 mm measured perpendicularly to an extension direction of the first channel.

6. The artificial heart valve of claim 2, wherein the stent further comprises second channel walls joined to the mesh, the second channel walls delimiting second channels for receiving portions of the prosthetic leaflet therein, and wherein the portions of the prosthetic leaflet are able to be inserted through the second channels and sutured to the second channel walls.

7. The artificial heart valve of claim 6, wherein the prosthetic leaflet comprises protruding portions, notches, a curved portion and a flat portion, the number of the protruding portions and the notches is both two, the two protruding portions contiguous with opposite ends of the flat portion, the two notches contiguous with opposite ends of the curved portion, each of the notches contiguous with a respective one of the protruding portions,
the curved portion configured to be inserted through the first channel and sutured to the first channel wall, the protruding portions configured to be inserted through the second channels and sutured to the second channel walls.

8. The artificial heart valve of claim 6, wherein the second channels extend along an axis of the stent, the second channels have a length of 1 mm to 10 mm measured in an extension direction of the second channels, and the second channels have a width of 0.1 mm to 2 mm measured perpendicularly to the extension direction of the second channels.

9. The artificial heart valve of any one of claims 2 to 8, wherein the prosthetic leaflet is sutured to the stent on an outer circumference of the stent.

10. The artificial heart valve of claim 9, wherein the suture comprises a suture body and two knot tails at respective opposite ends of the suture body, wherein when the prosthetic leaflet is sutured to the stent, the suture body and the knot tails are all located on the outer circumference of the stent.

11. The artificial heart valve of any one of claims 2 to 8, wherein the prosthetic leaflet is provided therein with a plurality of suture holes arranged in a circumferential direction of the prosthetic leaflet,
wherein when the prosthetic leaflet is sutured to the first channel wall, the suture is successively threaded through each of the suture holes and each time after the suture is threaded through one of the suture holes, the suture is connected to the first channel wall.

12. The artificial heart valve of claim 11, wherein at least two sutures are used, each of which is threaded through a predetermined number of suture holes.

13. The artificial heart valve of claim 11, wherein all the suture holes are distributed uniformly in the prosthetic leaflet, and all the suture holes are in symmetry in the prosthetic leaflet.

14. The artificial heart valve of claim 11, wherein the prosthetic leaflet comprises a curved portion, the curved portion is configured to be inserted through the first channel and sutured to the first channel wall, wherein adjacent suture holes are spaced at an interval of 0.1 mm to 3 mm and the suture holes are spaced at a distance of 0.1 mm to 5 mm from an edge of the curved portion, and wherein the suture holes have a diameter of 0.1 mm to 2 mm.
